# EUROPÄISCHE PATENTSCHRIFT

(11) **EP 3 323 347 B1**
(45) Veröffentlichungstag und Bekanntmachung des Hinweises auf die Patenterteilung: **16.04.2025**
(21) Anmeldenummer: 18150957.1
(22) Anmeldetag: 10.01.2018
(51) Int. Cl.: A61B 6/12, A61B 6/00, G06T 11/00

(54) **BILDUNTERSTÜTZUNG WÄHREND EINES MINIMALINVASIVEN CHIRURGISCHEN EINGRIFFS**
IMAGE-BASED SUPPORT DURING A SURGICAL MINIMALLY INVASIVE PROCEDURE
SOUTIEN BASÉ SUR L'IMAGE LORS D'UNE PROCÉDURE CHIRURGICALE MINI-INVASIVE

(30) Priorität: 02.03.2017 DE 102017203438
(43) Veröffentlichungstag der Anmeldung: 23.05.2018
(73) Patentinhaber: Siemens Healthineers AG, 91301 Forchheim (DE)
(72) Erfinder: Gemmel, Alexander, 91056 Erlangen (DE); Kreher, Björn, 91094 Bräuningshof (DE); Kleinszig, Gerhard, 91301 Forchheim (DE); Weiten, Markus, 90491 Nürnberg (DE)
(74) Vertreter: Siemens Healthineers Patent Attorneys

(56) Entgegenhaltungen:
- EP-A1- 3 127 485
- DE-A1- 102009 037 251
- DE-A1- 102009 049 818
- DE-A1- 102011 083 703
- ZHANG HUA ET AL: "Iterative Reconstruction for X-Ray Computed Tomography Using Prior-Image Induced Nonlocal Regularization", IEEE TRANSACTIONS ON BIOMEDICAL ENGINEERING, IEEE SERVICE CENTER, PISCATAWAY, NJ, USA, vol. 61, no. 9, 2 September 2014 (2014-09-02), pages 2367 - 2378, XP011556624, ISSN: 0018-9294, [retrieved on 20140818], DOI: 10.1109/TBME.2013.2287244

## Beschreibung

Die Erfindung betrifft eine Röntgeneinrichtung, ein Computerprogramm und einen elektronisch lesbaren Datenträger.

Bei minimalinvasiven chirurgischen Eingriffen, beispielsweise in der Traumatologie, müssen die entsprechenden medizinischen Instrumente, beispielsweise Werkzeuge und/oder Implantate, minimalinvasiv mit hoher Genauigkeit, beispielsweise am Knochen, positioniert werden. Dazu wurde vorgeschlagen, die korrekte Platzierung/Bewegung mithilfe einer Röntgeneinrichtung, beispielsweise einer mobilen Röntgeneinrichtung mit einem C-Bogen, zu überwachen. Problematisch hierbei ist jedoch, dass es sich bei den zweidimensionalen Röntgenbildern um Projektionsbilder handelt, die keinen eindeutigen Aufschluss über die dreidimensionale Struktur der dargestellten Objekte geben. Mithin ist es bekannt, dass die den Eingriff durchführende Person, insbesondere ein Arzt, Röntgenbilder aus mehreren Projektionsrichtungen aufnimmt, um alle wesentlichen Aspekte beurteilen zu können. Dabei kann es jedoch sowohl beim Justieren der medizinischen Instrumente als auch beim Anfahren neuer Aufnahmegeometrien mit der Röntgeneinrichtung, beispielsweise einem C-Bogen, zu ungewollten Bewegungen zwischen dem medizinischen Instrument und der Anatomie im Eingriffsbereich kommen. Das hat zur Konsequenz, dass die den Eingriff durchführende Person die verschiedenen Aufnahmegeometrien, beispielsweise C-Bogen-Positionen, mehrfach anfahren muss, um die korrekte Positionierung des medizinischen Instruments sicherzustellen.

In der Praxis wechselt also die den Eingriff durchführende Person die Aufnahmegeometrie so lange, bis sie sich in der Positionierung des medizinischen Instruments sicher ist. Stehen dreidimensionale Wiedergaben des Eingriffsbereichs zur Verfügung, beispielsweise Schichtbilder aus der Magnetresonanz, der Computertomographie oder dergleichen, wurde auch bereits vorgeschlagen, das mittels eines Positionsbestimmungssystems nachverfolgte medizinische Instrument nach einer erfolgreichen räumlichen Registrierung innerhalb eines derartigen präoperativen dreidimensionalen Bilddatensatzes darzustellen. Derartige Positionsbestimmungssysteme, die auch als Nachverfolgungssysteme bzw. Trackingsysteme bekannt sind, können beispielsweise elektromagnetisch, mittels optischer Marker und dergleichen arbeiten, wobei auch vorgeschlagen wurde, Marker im Röntgenbild zu nutzen. So kann das medizinische Instrument im dreidimensionalen Bilddatensatz aus einer beliebigen Blickrichtung dargestellt werden. Problematisch hierbei ist allerdings, dass die dabei dargestellte Anatomie im Eingriffsbereich aufgrund des Eingriffs mehr oder weniger stark von der tatsächlichen, aktuellen Anatomie im Eingriffsbereich abweichen kann.

In EP 3 127 485 A1 wird eine Röntgeneinrichtung beschrieben, welche während eines minimalinvasiven Eingriffs mit einem Instrument mehrere Bilder aus unterschiedlichen Projektionsrichtungen aufnimmt, wobei anhand dieser Bilder in einem iterativen Prozess eine 3D-Volumenrekonstruktion vorgenommen wird. Mithilfe des 3D-Volumens ist anschließend eine Reprojektion möglich.

Aus DE 10 2009 049 818 A1 ist ein Verfahren zur Ermittlung der Projektionsgeometrie einer Röntgenanlage bekannt. Dabei wird ein Röntgenbild eines Objekts erzeugt, welches in einen Patienten eingebracht ist, wobei eine charakteristische Dimension des Objekts gesucht und diese mit einer tatsächlichen, dreidimensionalen charakteristischen Dimension verglichen wird, um die Projektionsgeometrie zu ermitteln. Bei einer schwenkbaren Röntgenanlage können Projektionsgeometrien für verschiedene Schwenkwinkel ermittelt werden, um eine dreidimensionale, verbesserte Rekonstruktion des Objektes zu berechnen, nachdem die Projektionsgeometrien genauer bekannt sind.

DE 10 2009 037251 A1 offenbart ein Verfahren zum Erzeugen von 3D-Bilddaten eines Körpers mittels Durchleuchtung, wobei der Körper zumindest ein für die Durchleuchtung nahezu undurchlässiges Objekt enthält. Dabei werden mittels Durchleuchtung aus verschiedenen Blickrichtungen 2D-Bilder des Körpers erzeugt, die als Projektion des Objekts ein Objektbild enthalten. Anschließend wird zum Objekt ein Modell gewählt, welches das Objekt beschreibende Daten enthält, und zu jedem Objektbild wird ein die Projektion des Modells auf das 2D-Bild darstellendes Modellbild ermittelt und in das zugehörige Objektbild eingepasst. Im Bereich des Modellbildes werden dann vorhandene Bilddaten modifiziert, wobei aus den modifizierten 2D-Bildern 3D-Bilddaten rekonstruiert werden.

Der Erfindung liegt daher die Aufgabe zugrunde, eine Röntgeneinrichtung anzugeben, mit welcher ein demgegenüber verbessertes Bildunterstützungsverfahren für einen minimalinvasiven Eingriff durchführende Personen durchführbar ist.

Zur Lösung dieser Aufgabe werden Röntgeneinrichtungen gemäß

Anspruch 1 und gemäß Anspruch 2 vorgeschlagen. Darüber hinaus wird ein Computerprogramm gemäss Anspruch 11, sowie ein elektronisch lesbarer Datenträger gemäss Anspruch 12 vorgeschlagen.

Zusammenfassung der Offenbarung

Methoden zur iterativen Rekonstruktion (oft auch: algebraische Rekonstruktion) dreidimensionaler Datensätze aus zweidimensionalen Projektionsbildern sind im Stand der Technik bereits weitgehend bekannt. Bei derartigen iterativen Rekonstruktionsverfahren wird letztlich ein dreidimensionales Modell - der dreidimensionale Modelldatensatz - berechnet, welches die aufgenommenen Röntgenbilder (Projektionsbilder) erklärt. Dabei werden während der iterativen Rekonstruktion aus dem zu bestimmenden dreidimensionalen Modelldatensatz verschiedene virtuelle Vergleichsbilder simuliert und mit den aufgenommenen Röntgenbildern verglichen. Der dreidimensionale Modelldatensatz wird dann iterativ optimiert, indem der Fehler zwischen den simulierten Vergleichsbildern und den aufgenommenen Röntgenbildern minimiert wird. Ein derartiges iteratives Rekonstruktionsverfahren kann auch mit wenigen Röntgenbildern durchgeführt werden, also bei dünn besetzter Abdeckung hinsichtlich der Projektionsrichtungen, wobei jedoch dann eine Unterbestimmung vorliegt und der resultierende dreidimensionale Modelldatensatz die Anatomie für viele Anwendungen nur ungenügend beschreibt.

Hinsichtlich der vorliegenden Offenbarung wurde jedoch erkannt, dass ein aus den Röntgenbildern entstehender dreidimensionaler Modelldatensatz zwar insgesamt unterbestimmt und äußerst ungenau ist, aber gerade für die Aufnahmegeometrien, in denen die Röntgenbilder aufgenommen wurden, die optimale Qualität aufweist. Im hier beschriebenen Anwendungsfall stellen diese üblicherweise von der den Eingriff durchführenden Person eingriffsspezifisch gewählten Aufnahmegeometrien jedoch die relevanten Projektionsrichtungen dar, in denen die Person Informationen wiedergegeben haben möchte, beispielsweise die Position von Objekten relativ zur Anatomie. Bedenkt man nun zusätzlich, dass dreidimensionale Modellobjekte, wie beispielsweise ein Instrumentenmodell des verwendeten, nachverfolgten medizinischen Instruments, in einem derartigen dreidimensionalen Modelldatensatz positioniert werden können, ergibt sich, dass für ein derart durch Hinzufügen einer Zusatzinformation ergänztes dreidimensionales Modell des Eingriffsbereichs und die Aufnahmegeometrien, also Projektionsrichtungen, für die ein aufgenommenes Röntgenbild bei der iterativen Rekonstruktion berücksichtigt wurde, ein nahezu perfektes simuliertes Röntgenbild, also das wenigstens eine Unterstützungsbild, ermittelt werden kann. Es sei noch angemerkt, dass die Aufnahmegeometrien nicht zwangsläufig von der den Eingriff durchführenden Person gewählt sein müssen, sondern es auch denkbar ist, diese wenigstens teilweise, beispielsweise durch Auswertung eines dreidimensionalen präoperativen Bilddatensatzes, automatisch zu bestimmen.

Der den Eingriff durchführenden Person, beispielsweise einem behandelnden Arzt, wird mithin die Möglichkeit gegeben, bereits aufgenommene Röntgenbilder neu simulieren zu lassen, um Modellobjekte, beispielsweise das medizinische Instrument oder hinzugefügte geometrische Messobjekte, in einem dreidimensionalen Kontext, beispielsweise hinsichtlich der Verdeckung, dargestellt zu erhalten. Wesentlich ist hierbei, dass bei der Reprojektion (oft auch als Vorwärtsprojektion oder Simulation einer Röntgenaufnahme bezeichnet) genau die Aufnahmegeometrien verwendet werden, für die das dreidimensionale Modell optimiert ist.

Dabei kann vorgesehen ein, dass weniger als 10, insbesondere weniger als 5, eingriffsspezifisch gewählte Aufnahmegeometrien verwendet werden. Es hat sich in der Praxis gezeigt, dass während eines minimalinvasiven Eingriffs von der durchführenden Person meist nur etwa 2 bis 4 unterschiedliche Aufnahmegeometrien, also Projektionsrichtungen, ausgewählt werden, um die gewollten Informationen wiederzugeben. Obwohl das hieraus rekonstruierte dreidimensionale Modell bzw. konkret der dreidimensionale Modelldatensatz für andere Projektionsrichtungen ungenau ist, ist er doch genau auf diese gewählten, eingriffsspezifischen Projektionsrichtungen hin optimiert und liefert hervorragende Ergebnisse, ohne dass ein erneutes Anfahren der Aufnahmegeometrie mit nachfolgender Aufnahme eines aktuellen Röntgenbilds notwendig wäre.

Zusammenfassend schlägt die Offenbarung also vor, die während eines Eingriffs aufgenommenen zweidimensionalen Röntgenbilder in Zusammenhang zu bringen, um einen ungenauen bzw. unvollständigen dreidimensionalen Modelldatensatz der zugrunde liegenden Anatomie im Eingriffsbereich zu bestimmen, bei dem im Gegensatz zu einem hinreichend genauen, vollständigen dreidimensionalen Modelldatensatz die erwartete Bildqualität nur für simulierte Projektionsbilder ausreichend ist, deren Aufnahmegeometrie der eines akquirierten, bei der iterativen Rekonstruktion berücksichtigten Röntgenbildes entspricht. Mithin lassen sich Unterstützungsbilder für die Person erzeugen, ohne dass speziell Röntgenbilder aufgenommen werden müssen. Dies ermöglicht eine signifikante Reduzierung der Zeit des minimalinvasiven Eingriffs als auch der verabreichten Strahlendosis insbesondere dann, wenn ein tatsächlich röntgentechnisch erfasstes Modellobjekt, insbesondere das medizinische Instrument, anderweitig in seiner Position erfasst werden kann.

Die Verwendung des dreidimensionalen Modelldatensatzes weist weitere Vorteile im Vergleich zu einer gedachten Lösung auf, in der einfach nur das durch die Zusatzinformation hinzugefügte Modellobjekt vorwärtsprojiziert und in die Röntgenbilder eingeblendet wird. Zum einen wird dadurch, dass die iterative Rekonstruktion basierend auf mehreren Röntgenbildern durchgeführt wird, eine kleinere, aufgrund von beispielsweise Ungenauigkeiten in der Bestimmung der Aufnahmegeometrien oder kleinen Bewegungen des Patienten auftretende, Verschiebung der Anatomie zwischen der Aufnahme dieser Röntgenbilder automatisch ausgeglichen, so dass ein konsistentes Gesamtbild entsteht. Zum anderen aber werden anatomische Objekte zumindest prinzipiell im dreidimensionalen Modelldatensatz abgebildet, so dass mit der Hinzufügung einer Zusatzinformation in den dreidimensionalen Modelldatensatz auch die Anordnung relativ zu diesen anatomischen Objekten gegeben ist, mithin Verdeckungseffekte korrekt wiedergegeben werden können.

In einem Beispiel ist vorgesehen, dass als eine Zusatzinformation ein dreidimensionales Instrumentenmodell auf Grundlage einer aktuellen Positionsinformation des Instruments im Eingriffsbereich in den Modelldatensatz eingefügt wird und die Projektion des Instrumentenmodells in den Aufnahmegeometrien zeigende Unterstützungsbilder ermittelt und angezeigt werden. Ist also eine dreidimensionale Positionsinformation des Instruments, die selbstverständlich auch eine Orientierungsinformation neben einer reinen Ortsangabe umfassen kann, ermittelbar, kann ein entsprechendes Instrumentenmodell als Zusatzinformation in den dreidimensionalen Modelldatensatz mit der aktuellen Position und Orientierung eingefügt werden. Derartige Instrumentenmodelle lassen sich leicht beispielsweise mittels aus Datenbanken bekannten Abmessungen und Materialeigenschaften des medizinischen Instruments, welches beispielsweise ein Werkzeug und/oder ein Implantat sein kann, ableiten. Ohne Aufnahme von Röntgenbildern oder zumindest mit reduzierter Anzahl von Röntgenbildern werden der Person mithin Unterstützungsbilder bereitgestellt, die das Instrument an seiner aktuellen Position relativ zur Anatomie im Eingriffsbereich zeigen. Dies ermöglicht eine hochgenaue, zeiteffiziente und verlässliche Überwachung der Instrumentenpositionierung.

Vorzugsweise kann die Positionsinformation von einem mit der Röntgeneinrichtung registrierten Positionsbestimmungssystem und/oder aus einem zweidimensionalen Lokalisierungsbild der Röntgeneinrichtung ermittelt werden. Eine erste Möglichkeit zur Ermittlung der dreidimensionalen Positionsinformation ist also die Verwendung eines Positionsbestimmungssystems. Ein derartiges Positionsbestimmungssystem kann beispielsweise als elektromagnetisches Positionsbestimmungssystem oder optisches Positionsbestimmungssystem ausgebildet sein. Derartige Positionsbestimmungssysteme wurden im Stand der Technik bereits beschrieben, genau wie Möglichkeiten zur Registrierung von Bilddaten bzw. Koordinatensystemen von Bildgebungseinrichtungen mit solchen Positionsbestimmungssystemen. Zweckmäßig kann es im Rahmen der vorliegenden Erfindung jedoch auch sein, wenn ein zweidimensionales Lokalisierungsbild der Röntgeneinrichtung verwendet wird, um die Positionsinformation zu ermitteln. Dabei ist es beispielsweise denkbar, die dreidimensionale Position durch spezielle Röntgenmarker, die insbesondere an dem Instrument angeordnet werden können, zu ermitteln. Beispielsweise können hier Schlussfolgerungen aus der Darstellungsgröße gezogen werden. Eine vorteilhafte Ausgestaltung sieht auch vor, dass eine dreidimensionale Positionsinformation aus der Formgebung des Instruments und der entsprechenden Darstellung im Lokalisierungsbild bestimmt wird. Es kann mithin zusammenfassend vorgesehen sein, dass bei Verwendung eines einzigen Lokalisierungsbildes die Ermittlung der dreidimensionalen Positionsinformation aufgrund wenigstens eines Markers und/oder der Formgebung des Instruments erfolgt. Wird die Röntgeneinrichtung selbst genutzt, um die dreidimensionale Positionsinformation des Instruments zu bestimmen, entfallen vorteilhafterweise zusätzliche Geräte und zusätzlicher Aufwand, beispielsweise zur Registrierung.

Dabei sei an dieser Stelle noch angemerkt, dass dann, wenn eine der eingriffsspezifischen Aufnahmegeometrien genutzt wird, das Lokalisierungsbild selbstverständlich auch als ein Röntgenbild bzw. ein aktualisiertes Röntgenbild herangezogen werden kann, insbesondere auch, um den dreidimensionalen Modelldatensatz zu aktualisieren, worauf im Folgenden noch näher eingegangen werden wird.

Zweckmäßig ist es im Kontext eines Instrumentenmodells als Zusatzinformation auch, wenn bei bereits bei Aufnahme wenigstens eines Teils der Röntgenbilder im Eingriffsbereich befindlichen Instrument dieses in den Röntgenbildern und/oder in dem Modelldatensatz segmentiert und entfernt wird. Werden die Röntgenbilder, aus denen der dreidimensionale Modelldatensatz berechnet wird, mithin schon bei im Eingriffsbereich befindlichem Instrument aufgenommen, erscheint dann, wenn das Instrument durch Röntgenstrahlung sichtbar ist, das Instrument auch in dem dreidimensionalen Modelldatensatz, was unerwünscht ist, da es zu Irritierungen des Benutzers führen kann, mehrere Instrumente zu sehen. Daher werden zweckmäßigerweise Methoden der Bildverarbeitung genutzt, um das Instrument in den Röntgenbildern oder auch im Modelldatensatz zu detektieren, zu segmentieren und zu entfernen.

Beispielsweise ist ein Workflow denkbar, in dem die Person zunächst die Röntgenbilder aus den verschiedenen Projektionsrichtungen akquiriert, um danach die Lage des getrackten Instruments zu verändern. Dann kann mittels der erfindungsgemäßen Röntgeneinrichtung die neue Lage des Instruments auf den Unterstützungsbildern, also simulierten Röntgenbildern, für die zuvor angefahrenen Projektionsrichtungen aktualisiert und dargestellt werden.

Ein weiteres vorteilhaftes Anwendungsgebiet der vorliegenden Erfindung ist die Vornahme von Messungen bzw. sonstigen Planungen im Eingriffsbereich. Denn oft entsteht der Wunsch, beispielsweise den Abstand zwischen bestimmten anatomischen Strukturen, deren Durchmesser, Fläche und dergleichen zu ermitteln, beispielsweise, um ein geeignetes medizinisches Instrument, beispielsweise eine Schraube als Implantat, auswählen zu können.

Ein weiteres Beispiel sieht vor, dass zur Ermittlung wenigstens einer der wenigstens einen Zusatzinformation zu wenigstens zwei in einem der Röntgenbilder oder einem Unterstützungsbild als Auswahlbild benutzerseitig gewählten Auswahlpunkten jeweils eine dreidimensionale Position des Auswahlpunktes in dem Modelldatensatz bestimmt wird, indem entlang eines in der Aufnahmegeometrie des Auswahlbildes, in dem die Auswahlpunkte gewählt wurden, definierten Strahles, der den Auswahlpunkt durchquert, nach einem Intensitätssprung in wenigstens einer Suchrichtung in dem Modelldatensatz gesucht wird, dessen Position als dreidimensionale Position des Auswahlpunktes gewählt wird, und wenigstens ein mit den Auswahlpunkten als Zusatzinformation in einer anderen Aufnahmegeometrie als der des Auswahlbildes, in dem die Auswahlpunkte gewählt wurden, reprojiziertes Unterstützungsbild ermittelt wird.

Mit anderen Worten kann der Benutzer, insbesondere also die Person, in einem Auswahlbild, welches eines der Röntgenbilder, aber auch ein Unterstützungsbild sein kann, Auswahlpunkte wählen, die beispielsweise eine Strecke oder eine Fläche definieren können. Das Vorliegen des dreidimensionalen Modelldatensatzes ermöglicht es nun, zumindest Vermutungen darüber anzustellen, welche dreidimensionale Position des Auswahlpunktes seitens des Benutzers gemeint gewesen sein könnte. Denn die Aufnahmegeometrie beschreibt den Verlauf des Strahls, der am Auswahlpunkt endet, so dass entlang dieses Strahls nach Intensitätssprüngen gesucht werden kann, die ausgezeichnete dreidimensionale Stellen anzeigen, welche als dreidimensionaler Auswahlpunkt gemeint gewesen sein könnten. Ein solcher Suchprozess kann auch als "Picking" bezeichnet werden und wird für jeden der Auswahlpunkte durchgeführt. Ergebnis sind dreidimensionale Positionen für die Auswahlpunkte, die genutzt werden, um die vermuteten Auswahlpunkte auch in Unterstützungsbildern wenigstens einer anderen Aufnahmegeometrie anzeigen zu können, so dass die Person überprüfen kann, ob tatsächlich der korrekte Auswahlpunkt in drei Dimensionen aufgefunden wurde.

In einer einfach zu realisierenden Ausgestaltung kann vorgesehen sein, dass die Suchrichtung senkrecht zu der Strahlrichtung gewählt wird. Nachdem, wie bereits dargelegt wurde, der dreidimensionale Modelldatensatz am genauesten hinsichtlich der Aufnahmegeometrien ist, in denen ja auch Röntgenbilder vorliegen, hat es sich alternativ oder zusätzlich als zweckmäßig erwiesen, als Suchrichtungen Strahlrichtungen der Aufnahmegeometrie wenigstens des Unterstützungsbildes oder des Auswahlbildes, bevorzugt aller Aufnahmegeometrien, durch einen aktuell untersuchten Punkt des Strahls verwendet werden. So wird die Analyse im dreidimensionalen Modelldatensatz dort vorgenommen, wo die verlässlichste Information vorliegt.

In einem weiteren Beispiel kann vorgesehen sein, dass die dreidimensionale Position des Auswahlpunktes in Abhängigkeit einer benutzerseitigen Verschiebung des Auswahlpunktes in dem Unterstützungsbild entlang des in das Unterstützungsbild projizierten zugeordneten Strahls angepasst wird. Wird ein Auswahlpunkt in einem Auswahlbild definiert, bilden sich die möglichen dreidimensionalen Lagen, wenn keine andere Information vorliegt, als Linie in einem Unterstützungsbild der Aufnahmegeometrie der anderen Projektionsrichtung ab. Wird nun die Bestimmung der dreidimensionalen Position des Auswahlpunktes durch Suchen von Intensitätssprüngen entlang des Strahls gemäß dem Auswahlbild als erste Annahme verstanden, kann dem Benutzer die Wahl gelassen werden, diese dreidimensionale Lage dann noch zu verändern, indem der in dem Unterstützungsbild dargestellte Auswahlpunkt verschoben wird. Die Verschiebung wird dann auf die genannte Linie in dem Unterstützungsbild eingeschränkt. Wurden mehrere Intensitätssprünge entlang des Strahls festgestellt und beispielsweise der erste und/oder betragsmäßig größte als die dreidimensionale Position des Auswahlpunktes markierend angenommen, kann auch vorgesehen sein, bei Verschiebungen die Positionen der anderen Intensitätssprünge gezielt anzubieten. So kann die Person nicht nur überprüfen, ob die dreidimensionale Position des Auswahlpunktes automatisiert korrekt gewählt wurde, sondern diese, falls dies nicht der Fall ist, hervorragend unterstützt geeignet anpassen.

Vorzugsweise kann auf Basis der dreidimensionalen Positionen der Auswahlpunkte wenigstens ein Maß einer durch die Auswahlpunkte aufgespannten geometrischen Form ermittelt und ausgegeben werden, insbesondere im Fall von zwei Auswahlpunkten eine Distanz zwischen den Auswahlpunkten. Letztlich ist damit insgesamt ein Workflow gegeben, der eine Vermessung von Abständen im dreidimensionalen Raum (statt nur im zweidimensionalen Raum eines Röntgenbildes) erlaubt. Dabei wird der Benutzer gezielt bei der korrekten Definition dreidimensionaler Punkte unterstützt, indem das Hintergrundwissen des Modelldatensatzes zumindest bezüglich der Projektionsrichtungen der eingriffsspezifischen Aufnahmegeometrien genutzt wird.

Ein sich hier ergebender Workflow könnte beispielsweise umfassen, dass nach der Aufnahme der Röntgenbilder in den verschiedenen Projektionsrichtungen die Person, beispielsweise mittels einer Maus, auf einem Röntgenbild zwei Auswahlpunkte definiert, die wiederum eine Linie auf dem Röntgenbild definieren. Die die Erfindung durchführende Recheneinrichtung, beispielsweise ein Bildsystem einer Röntgeneinrichtung, berechnet für beide Auswahlpunkte die wahrscheinlichsten dreidimensionalen Positionen, insbesondere wie beschrieben von Intensitätsübergängen im dreidimensionalen Modelldatensatz entlang des Strahlenverlaufs, woraufhin die durch die aufgefundenen 3D-Koordinaten der Auswahlpunkte definierte Linie (samt den Auswahlpunkten) auf Unterstützungsbildern, also simulierten Röntgenbildern, der anderen zuvor angefahrenen Aufnahmegeometrien dargestellt wird. Die Person überprüft anhand der Unterstützungsbilder die korrekte Positionierung und korrigiert bei Bedarf. Dann kann die Länge der Linie als Distanz der Auswahlpunkte von der das Verfahren durchführenden Recheneinrichtung, insbesondere dem Bildsystem der Röntgeneinrichtung, berechnet und der Person mitgeteilt werden.

Ein Beispiel sieht ferner vor, dass der Modelldatensatz bei Neuaufnahme eines aktuellen Röntgenbildes in wenigstens einer der Aufnahmegeometrien aktualisiert wird, insbesondere nach einer 2D-3D-Registrierung des Modelldatensatzes und des aktuellen Röntgenbildes. Auf diese Weise können Ungenauigkeiten durch Sensorik, Patientenbewegungen und dergleichen minimiert werden, indem eine 2D-3D-Registrierung erfolgt, bevor die Berücksichtigung neuer, aktueller Röntgenbilder im Modelldatensatz stattfindet. Es sei an dieser Stelle noch angemerkt, dass zur Durchführung des Verfahrens, welches nicht Teil der Erfindung ist, selbstverständlich die Röntgeneinrichtung bzw. entsprechend bewegbare Komponenten in der Lage sein müssen, ihre Einstellungen und somit die Aufnahmegeometrien automatisch zu bestimmen, was jedoch bei vielen modernen Röntgeneinrichtungen ohnehin möglich ist.

Insbesondere kann die Röntgeneinrichtung einen C-Bogen aufweisen, an dem sich gegenüberliegend ein Röntgenstrahler und ein Röntgendetektor angeordnet sind. Ferner kann vorgesehen sein, dass die Steuereinrichtung als Teil eines Bildsystems realisiert ist. Die Steuereinrichtung kann neben einer Aufnahmesteuereinheit zur Steuerung des Aufnahmebetriebs der Röntgeneinrichtung eine Rekonstruktionseinheit zur Ermittlung des dreidimensionalen Modelldatensatzes und eine Reprojektionseinheit zur Ermittlung von Unterstützungsbildern umfassen.

Ein Computerprogramm ist direkt in einen Speicher einer Steuereinrichtung einer Röntgeneinrichtung ladbar und weist Programmmittel auf, um die Schritte eines hierin beschriebenen Verfahrens auszuführen, wenn das Computerprogramm in der Steuereinrichtung der Röntgeneinrichtung ausgeführt wird. Das Computerprogramm kann auf einem elektronisch lesbaren Datenträger abgespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Steuereinrichtung einer Röntgeneinrichtung ein hierin beschriebenes Verfahren durchführen. Bei dem Datenträger kann es sich um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels eines Verfahrens, welches von der Steuereinrichtung einer erfindungsgemäßen Röntgeneinrichtung durchgeführt wird,
- Fig. 2: eine Prinzipskizze zur Aufnahme mit unterschiedlichen Aufnahmegeometrien und zur Ermittlung des dreidimensionalen Modelldatensatzes,
- Fig. 3: einen Ablaufplan zu einer ersten Anwendung des Verfahrens,
- Fig. 4: eine Prinzipskizze zur Erzeugung von Unterstützungsbildern,
- Fig. 5: einen Ablaufplan zu einer zweiten Anwendung des Verfahrens,
- Fig. 6: eine Prinzipskizze zum Auffinden einer dreidimensionalen Position eines Auswahlpunktes, und
- Fig. 7: eine erfindungsgemäße Röntgeneinrichtung.

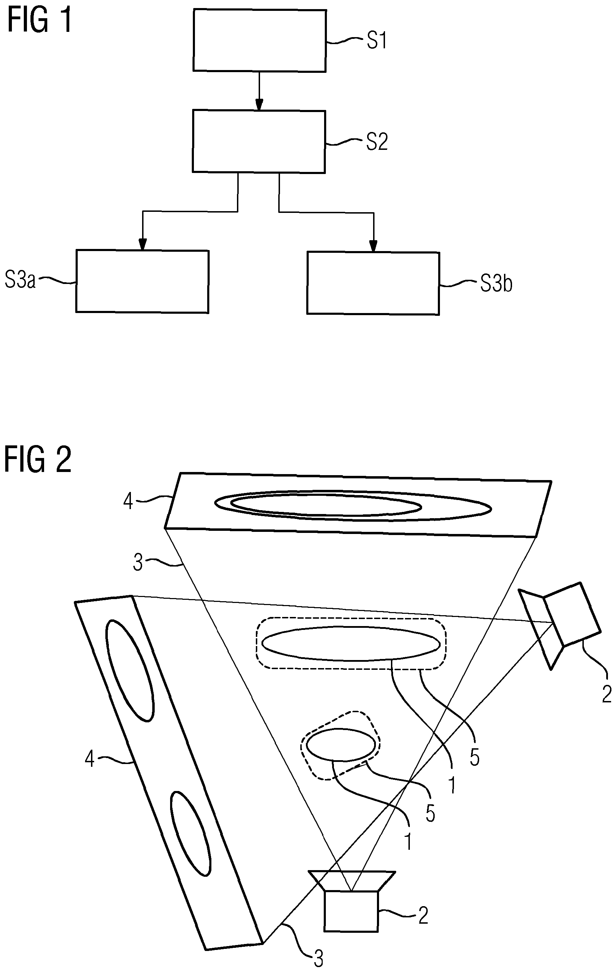

Fig. 1 zeigt einen prinzipiellen Ablaufplan eines Ausführungsbeispiels eines Verfahrens, welches von der Steuereinrichtung einer erfindungsgemäßen Röntgeneinrichtung durchgeführt wird. Dabei soll eine einen minimalinvasiven Eingriff durchführende Person durch Bilder unterstützt werden. Um dies zu ermöglichen, ist zunächst in einem Schritt S1 vorgesehen, dass mittels einer Röntgeneinrichtung in von der Person gewählten, eingriffsspezifischen, unterschiedlichen Projektionsrichtungen entsprechenden Aufnahmegeometrien zweidimensionale Röntgenbilder mit einer Röntgeneinrichtung aufgenommen werden. Dabei handelt es sich vorliegend um eine Röntgeneinrichtung mit einem C-Bogen, der auch an einem Eingriffsort flexibel positioniert werden kann, um die Röntgenbilder aufzunehmen. Wird ein Beispiel aus der Traumatologie verwendet, kann die Person beispielsweise die eingriffsspezifischen Aufnahmegeometrien so wählen, dass die korrekte Lage eines Implantats als medizinisches Instrument optimal beurteilt werden kann. Vorliegend werden beispielhaft zwei Röntgenbilder, mithin zwei eingriffsspezifische Aufnahmegeometrien betrachtet, wobei auch beispielsweise drei oder vier Röntgenbilder aus unterschiedlichen Projektionsrichtungen denkbar sind. Die Röntgenbilder werden vorliegend noch ohne das medizinische Instrument aufgenommen; in anderen Fällen ist vorgesehen, das medizinische Instrument nachher herauszurechnen.

Durch iterative Rekonstruktion wird in einem Schritt S2 aus den zweidimensionalen Röntgenbildern ein dreidimensionaler Modelldatensatz des in den Röntgenbildern abgebildeten Eingriffsbereichs des Patienten erzeugt. Dabei wird bewusst in Kauf genommen, dass der Modelldatensatz unterbestimmt ist und die dreidimensionale Anatomie im Eingriffsbereich nicht vollständig korrekt wiedergeben kann, nachdem es vorliegend ausreichend ist, bei einer Reprojektion in den Aufnahmegeometrien wieder korrekte Bilder zu erhalten, was der Fall ist, nachdem auf die möglichst genaue Wiedergabe der Röntgenbilder hin optimiert wird. Dies ist schematisch in Fig. 2 dargestellt. Gezeigt sind beispielhaft reale anatomische Strukturen 1 im Eingriffsbereich, wobei die hier betrachteten zwei Aufnahmegeometrien durch die Position des Röntgenstrahlers 2 sowie die jeweiligen Erfassungsbereiche 3 angedeutet sind. Gezeigt sind ferner schematisch die sich jeweils ergebenden Röntgenbilder 4, die projizierte Abbilder der anatomischen Strukturen 1 zeigen.

Wird durch iterative Rekonstruktion ein dreidimensionaler Modelldatensatz erzeugt, gibt dieser die realen anatomischen Strukturen 1 nicht vollständig korrekt wieder, sondern als unterbestimmte, hier nur äußerst schematisch angedeutete rekonstruierte Anatomieobjekte 5, die so beschaffen sind, dass bei Reprojektion aus dem dreidimensionalen Modelldatensatz die anatomischen Strukturen 1 in reprojizierten simulierten Röntgenbildern korrekt dargestellt werden. Eventuelle kleinere Abweichungen/Verschiebungen wurden durch den iterativen Rekonstruktionsprozess automatisch ausgemittelt, so dass ein konsistentes Gesamtbild entstanden ist.

Der so entstandene dreidimensionale Modelldatensatz des Schrittes S2 kann nun in verschiedenen Anwendungen genutzt werden, die vorliegend durch die Schritte S3a und S3b in Fig. 1 gekennzeichnet sind. Jede dieser Anwendungen involviert das Hinzufügen einer Zusatzinformation, insbesondere eines Zusatzobjekts, in den dreidimensionalen Modelldatensatz und eine Reprojektion, um ein Unterstützungsbild zu erhalten.

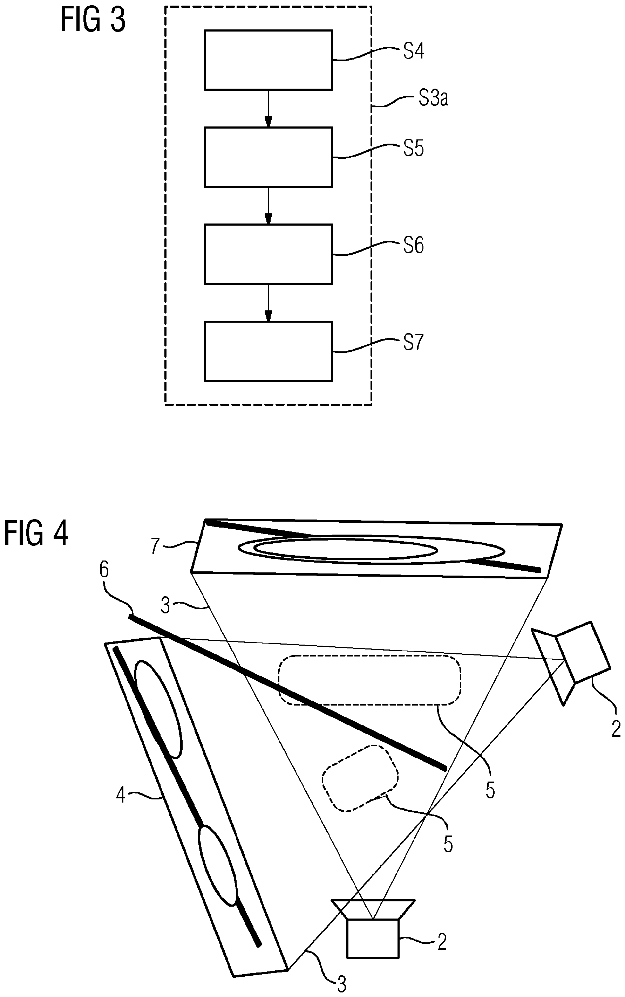

Fig. 3 erläutert eine erste Anwendung durch Darstellung entsprechender Unterschritte des Schrittes S3a genauer. Dort soll die aktuelle Lage eines verwendeten medizinischen Instruments, insbesondere eines Werkzeugs und/oder eines Implantats, bei zumindest teilweise vermiedener neuer Aufnahme von Röntgenbildern in Unterstützungsbildern der eingriffsspezifischen, somit vorbestimmten Aufnahmegeometrien dargestellt werden.

Dazu wird in einem Schritt S4 zunächst eine dreidimensionale Positionsinformation des medizinischen Instruments bestimmt. Diese enthält sowohl eine Ortsangabe als auch eine Orientierung. Zur Ermittlung der aktuellen dreidimensionalen Positionsinformation kann beispielsweise ein elektromagnetisches und/oder ein optisches Positionsbestimmungssystem verwendet werden. Denkbar ist es aber auch, die aktuelle Positionsinformation aus einem, insbesondere einem einzigen, Lokalisierungsbild mit der Röntgeneinrichtung zu ermitteln, wobei Marker oder bevorzugt die geometrische Formgebung des medizinischen Instruments genutzt werden, um aus dem einzigen zweidimensionalen Lokalisierungsbild auch eine dreidimensionale Lokalisierungsinformation abzuleiten. Ist das zweidimensionale Lokalisierungsbild in einer der Aufnahmegeometrien aufgenommen worden, bildet es mithin ein aktuelles Röntgenbild, und soll es zur Aktualisierung des dreidimensionalen Modelldatensatzes verwendet werden, wird zunächst eine 2D-3D-Registrierung mit dem dreidimensionalen Modelldatensatz durchgeführt. Die Verwendung eines Positionsbestimmungssystems hat den Vorteil, dass keine weitere Strahlenbelastung für den Patienten auftritt; die Verwendung eines Lokalisierungsbildes der Röntgeneinrichtung hat den Vorteil, dass keine weiteren Gerätschaften vorhanden sein müssen und dass jene weiteren Gerätschaften, konkret ein Positionsbestimmungssystem, nicht erst mit dem Koordinatensystem der Röntgeneinrichtung, insbesondere des C-Bogens, registriert werden müssen.

Aufgrund einer beispielsweise während eines Kalibrierungsschrittes hergestellten Registrierung bei Verwendung eines Positionsbestimmungssystems bzw. bei ohnehin vorhandenem Bezug bei Aufnahme eines Lokalisierungsbildes ist es nun möglich, in einem Schritt S5 dem dreidimensionalen Modelldatensatz als Zusatzinformation ein Instrumentenmodell zuzufügen, welches sich vorwiegend aus aus einer Datenbank abgerufenen geometrischen Abmessungen und Schwächungseigenschaften des Instruments ergibt.

In einem Schritt S6 ist es dann möglich, durch Reprojektion des ergänzten dreidimensionalen Modelldatensatzes Unterstützungsbilder als simulierte Röntgenbilder in den Aufnahmegeometrien zu erhalten, welche in einem Schritt S7 zur Anzeige gebracht werden können. Dieser Vorgang wird durch die Prinzipdarstellung der Fig. 4 nochmals näher erläutert. Zu erkennen sind wiederum die angedeuteten eingriffsspezifischen Aufnahmegeometrien (Position der Röntgenstrahler 2, Erfassungsbereiche 3) sowie die rekonstruierten Anatomieobjekte 5. Hinzugefügt ist ein Instrumentenmodell 6. Erfolgt nun eine Vorwärtsprojektion gemäß den Aufnahmegeometrien, werden Unterstützungsbilder 7 erhalten, die ein Abbild des medizinischen Instruments genauso wie der Anatomie im Eingriffsbereich enthalten.

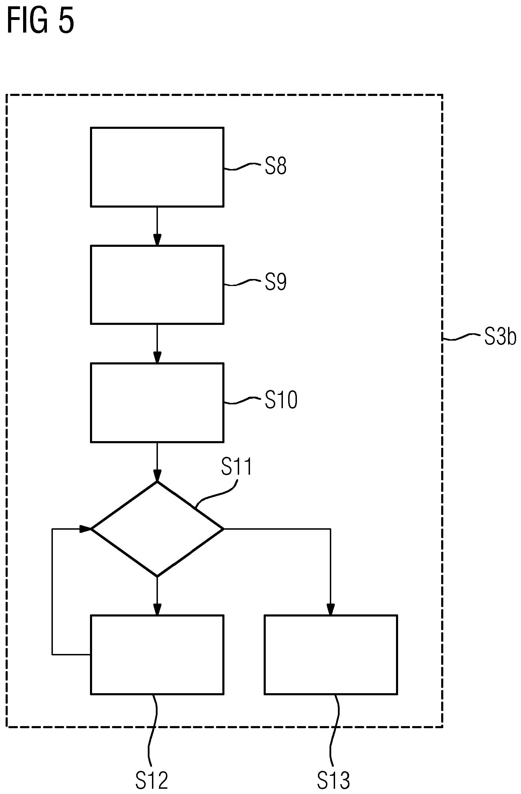

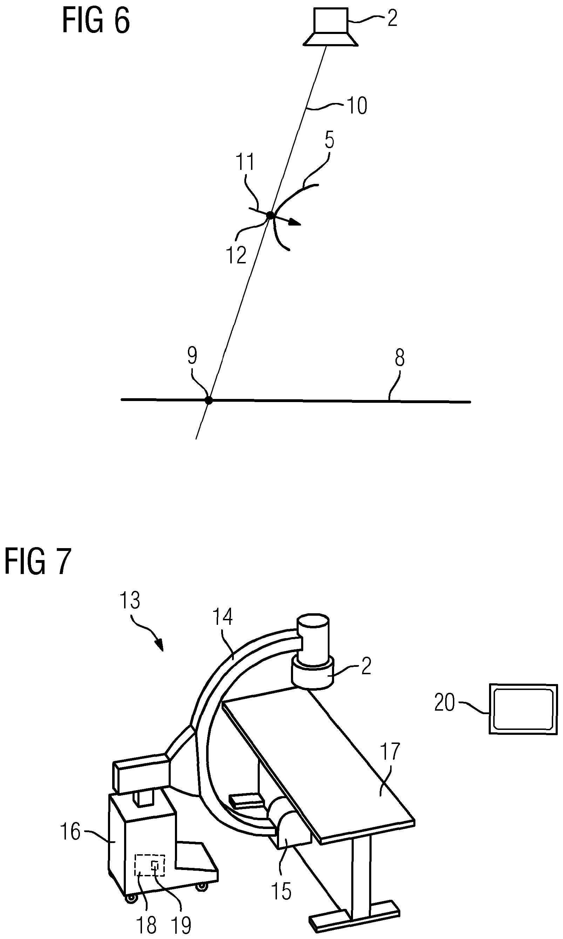

Eine weitere Anwendung zeigt Fig. 5 durch Darstellung von Unterschritten des Schrittes S3b. Dort ist vorgesehen, einen Abstand zwischen zwei Punkten im Eingriffsbereich zu vermessen. Daher markiert die Person zunächst im Schritt S8 in einem der Röntgenbilder 4 oder auch einem Unterstützungsbild 7 (zusammenfassend zur Vereinfachung als Auswahlbild bezeichnet) zwei Auswahlpunkte an entsprechenden (projizierten) Positionen. In einem Schritt S9 soll im Rahmen eines "Picking"-Prozesses nun abgeschätzt werden, welche dreidimensionale Position der Auswahlpunkte gemeint war, was mit Hilfe des dreidimensionalen Modelldatensatzes geschieht. Dies sei mit Hilfe von Fig. 6 näher erläutert, die einen im Auswahlbild 8 benutzerseitig markierten Auswahlpunkt 9 zeigt. Zu diesem Auswahlpunkt 9 gehört in der Aufnahmegeometrie des Auswahlbildes 8 ein von dem Röntgenstrahler 2 ausgehender Strahl 10, auf dem der Auswahlpunkt liegen muss. Mithin ist im Schritt S9 vorgesehen, entlang dieses Strahls 10 in wenigstens einer Suchrichtung 11 nach Intensitätssprüngen zu suchen, wobei ein solcher an der dreidimensionalen Position 12 durch den angedeuteten Rand des rekonstruierten Anatomieobjekts 5 gegeben ist. Die Suchrichtung 11 kann dabei als Strahlrichtung in der anderen Aufnahmegeometrie an der jeweiligen dreidimensionalen Position 12 entlang des Strahls 10 gewählt oder aber senkrecht zu dem Strahl 10 gewählt werden. Bei mehreren weiteren Aufnahmegeometrien werden bevorzugt deren aller Strahlrichtungen als Suchrichtung verwendet.

Der erste gefundene oder deutlichste Intensitätssprung markiert die dreidimensionale Position 12 des Auswahlpunktes 9, wie sie automatisch abgeschätzt wird. An dieser dreidimensionalen Position 12 wird nun als Zusatzinformation der Auswahlpunkt dem dreidimensionalen Modelldatensatz hinzugefügt und es erfolgt in Schritt S10 eine Reprojektion, also eine Vorwärtsprojektion, aus dem so ergänzten dreidimensionalen Modelldatensatz in der anderen Aufnahmegeometrie, um ein Unterstützungsbild zu erhalten, in dem ebenso eine Lage der jeweiligen Auswahlpunkte 9 zu erkennen ist.

In einem Schritt S11 kann die Person dann überprüfen, ob die Annahme der dreidimensionalen Position 12, wie sie sich in dem Unterstützungsbild widerspiegelt, korrekt ist. Ist dies nicht der Fall, kann sie über entsprechende Bedienmittel, beispielsweise eine Maus, den jeweiligen Auswahlpunkt 9 im Unterstützungsbild entlang der Projektion des Strahls 10 an die eigentlich gewollte Stelle verschieben. Die dreidimensionale Position 12 des Auswahlpunktes 9 wird entsprechend nachgeführt und angepasst. Nachdem dies in Schritt S12 geschehen ist, kann die Person im Schritt S11 erneut überprüfen, ob die korrekte dreidimensionale Lage der Auswahlpunkte 9 gegeben ist.

Wird die dreidimensionale Position 12 der Auswahlpunkte 9 bestätigt, wird in einem Schritt S13 automatisch seitens der das Verfahren durchführenden Recheneinrichtung, insbesondere einem Bildsystem einer Steuereinrichtung der Röntgeneinrichtung, der Abstand zwischen den Auswahlpunkten, der gesucht war, bestimmt und der Person zur Anzeige gebracht.

Fig. 7 zeigt schließlich eine Prinzipskizze einer erfindungsgemäßen Röntgeneinrichtung 13, die einen zumindest verschwenkbaren C-Bogen 14 umfasst, an dem sich gegenüberliegend der Röntgenstrahler 2 und ein Röntgendetektor 15 angeordnet sind. Der C-Bogen 14 ist an einer mobilen Einheit 16 angeordnet, um die Röntgeneinrichtung 13 im Eingriffsraum bewegen zu können und beispielsweise, wenn nötig, an einen entsprechenden Patiententisch 17 heranfahren zu können. Nur angedeutet ist die Steuereinrichtung 18, die den Betrieb der Röntgeneinrichtung 13 steuert. Durch die Steuereinrichtung 18 können aktuelle Positionen des C-Bogens 14 bzw. des Röntgendetektors 15/Röntgenstrahlers 2 und somit aktuell eingestellte Aufnahmegeometrien automatisch ermittelt werden. Sie ist vorliegend auch zur Durchführung des Verfahrens ausgebildet, wobei auch das Bildsystem 19 genutzt wird, mit welchem Berechnungen zur iterativen Rekonstruktion und zur Reprojektion besonders einfach durchgeführt werden können, beispielsweise mittels einer entsprechenden Rekonstruktionseinheit und einer entsprechenden Reprojektionseinheit. Im Eingriffsraum ist zudem eine Anzeigeeinrichtung 20 angeordnet, um die Unterstützungsbilder 7 und auch die Röntgenbilder 4 entsprechend anzeigen zu können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgeneinrichtung (13), aufweisend eine zur Durchführung eines Verfahrens zur Bildunterstützung einer einen minimalinvasiven Eingriff mit einem Instrument in einem Eingriffsbereich eines Patienten durchführenden Person ausgebildete Steuereinrichtung (18),
wobei mittels der Röntgeneinrichtung (13) in wenigstens zwei unterschiedliche Projektionsrichtungen realisierenden, eingriffsspezifisch, insbesondere durch die Person, gewählten Aufnahmegeometrien jeweils ein zweidimensionales Röntgenbild (4) des Eingriffsbereichs aufnehmbar ist,
wobei die Steuereinrichtung (18) dazu ausgebildet ist, durch iterative Rekonstruktion aus den Röntgenbildern (4) einen dreidimensionalen Modelldatensatz des Eingriffsgebiets zu rekonstruieren, wobei die Steuereinrichtung (18) weiterhin dazu ausgebildet ist wenigstens ein zweidimensionales Unterstützungsbild (7), welches einer der Aufnahmegeometrien entspricht, unter Einbeziehung wenigstens einer Zusatzinformation zu reprojizieren, um den um die Zusatzinformation ergänzten Modelldatensatz zu ermitteln und darzustellen, und
wobei die Steuereinrichtung (18) dazu eingerichtet ist, ein dreidimensionales Instrumentenmodell (6) als Zusatzinformation auf Grundlage einer aktuellen Positionsinformation des Instruments im Eingriffsbereich in den Modelldatensatz einzufügen und die Projektion des Instrumentenmodells (6) in den Aufnahmegeometrien zeigenden Untersuchungsbildern (7) (7) zu ermitteln und anzuzeigen.

2. Röntgeneinrichtung (13), aufweisend eine zur Durchführung eines Verfahrens zur Bildunterstützung einer einen minimalinvasiven Eingriff mit einem Instrument in einem Eingriffsbereich eines Patienten durchführenden Person ausgebildete Steuereinrichtung (18),
wobei mittels der Röntgeneinrichtung (13) in wenigstens zwei unterschiedliche Projektionsrichtungen realisierenden, eingriffsspezifisch, insbesondere durch die Person, gewählten Aufnahmegeometrien jeweils ein zweidimensionales Röntgenbild (4) des Eingriffsbereichs aufnehmbar ist,
wobei die Steuereinrichtung (18) dazu ausgebildet ist, durch iterative Rekonstruktion aus den Röntgenbildern (4) einen dreidimensionalen Modelldatensatz des Eingriffsgebiets zu rekonstruieren,
wobei die Steuereinrichtung (18) weiterhin dazu ausgebildet ist,
wenigstens ein zweidimensionales Unterstützungsbild (7), welches einer der Aufnahmegeometrien entspricht, unter Einbeziehung wenigstens einer Zusatzinformation reprojizieren, um den um die Zusatzinformation ergänzten Modelldatensatz zu ermitteln und darzustellen,
und
- wobei die Steuereinrichtung (18) dazu eingerichtet ist, wenigstens zwei, in einem der Röntgenbilder (4) oder einem Unterstützungsbild (7) als Auswahlbild (8) benutzerseitig gewählten Auswahlpunkten (9) jeweils eine dreidimensionale Position (12) des Auswahlpunktes (9) in dem Modelldatensatz zu bestimmen, indem entlang eines in der Aufnahmegeometrie des Auswahlbildes (8), in dem die Auswahlpunkte (9) gewählt wurden, definierten Strahles (10), der den Auswahlpunkt (9) durchquert, nach einem Intensitätssprung in wenigstens einer Suchrichtung (11) in dem Modelldatensatz gesucht wird, dessen Position als dreidimensionale Position (12) des Auswahlpunktes (9) gewählt wird, und
- wenigstens ein mit den Auswahlpunkten (9) als Zusatzinformation in einer anderen Aufnahmegeometrie als der des Auswahlbildes (8), in dem die Auswahlpunkte (9) gewählt wurden, reprojiziertes Unterstützungsbild (7) zu ermitteln.

3. Röntgeneinrichtung nach Anspruch 1 oder 2, wobei weniger als 10, insbesondere weniger als 5, eingriffsspezifisch gewählte Aufnahmegeometrien verwendet werden.

4. Röntgeneinrichtung nach einem der Ansprüche 1 bis 3, wobei die Positionsinformation von einem mit der Röntgeneinrichtung (13) registrierten Positionsbestimmungssystem und/oder aus einem zweidimensionalen Lokalisierungsbild der Röntgeneinrichtung (13) ermittelbar ist.

5. Röntgeneinrichtung nach Anspruch 4, wobei bei Verwendung eines einzigen Lokalisierungsbildes die Ermittlung der dreidimensionalen Positionsinformation aufgrund wenigstens eines Markers und/oder der Formgebung des Instruments erfolgt.

6. Röntgeneinrichtung nach einem der Ansprüche 1 bis 5, wobei die Steuereinrichtung (18) dazu eingerichtet ist, bei bereits bei Aufnahme wenigstens eines Teils der Röntgenbilder (4) im Eingriffsbereich befindlichen Instrument dieses in den Röntgenbildern (4) und/oder in dem Modelldatensatz zu segmentieren und zu entfernen.

7. Röntgeneinrichtung nach einem der Ansprüche 2 bis 6, wobei als Suchrichtungen (11) zu dem Strahl (10) senkrechte Richtungen und/oder Strahlrichtungen der Aufnahmegeometrie wenigstens des Unterstützungsbildes (7) oder Auswahlbildes (8), bevorzugt aller Aufnahmegeometrien, durch einen aktuell untersuchten Punkt des Strahls (10) verwendet werden.

8. Röntgeneinrichtung nach einem der Ansprüche 2 bis 7, wobei die Steuereinrichtung (18) dazu eingerichtet ist, die dreidimensionale Position (12) des Auswahlpunktes (9) in Abhängigkeit einer benutzerseitigen Verschiebung des projizierten Auswahlpunktes (9) in dem Unterstützungsbild (7) entlang des in das Unterstützungsbild (7) projizierten zugeordneten Strahls (10) anzupassen.

9. Röntgeneinrichtung nach einem der Ansprüche 2 bis 8, wobei auf Basis der dreidimensionalen Positionen (12) der Auswahlpunkte (9) wenigstens ein Maß einer durch die Auswahlpunkte (9) aufgespannten geometrischen Form ermittelbar und ausgebbar ist, insbesondere im Fall von zwei Auswahlpunkten (9) eine Distanz zwischen den Auswahlpunkten (9).

10. Röntgeneinrichtung nach einem der vorangehenden Ansprüche, wobei die Steuereinrichtung (18) dazu eingerichtet ist, den Modelldatensatz bei Neuaufnahme eines aktuellen Röntgenbildes (4) in wenigstens einer der Aufnahmegeometrien zu aktualisieren, insbesondere nach einer 2D-3D-Registrierung des Modelldatensatzes und des aktuellen Röntgenbildes (4).

11. Computerprogramm, welches zur Einrichtung einer Steuereinrichtung einer Röntgeneinrichtung nach einem der vorangehenden Ansprüche in einen Speicher der Steuereinrichtung ladbar ist.

12. Elektronisch lesbarer Datenträger, auf dem ein Computerprogramm nach Anspruch 11 gespeichert ist.

## Claims

1. X-ray apparatus (13), having a controller (18) embodied for carrying out a method of image support for a person carrying out a minimally invasive procedure with an instrument in a procedure site of a patient,
wherein one two-dimensional X-ray image (4) respectively of the procedure site is recorded by means of the X-ray apparatus (13) in at least two recording geometries implementing different projection directions and chosen specific to the procedure, in particular by the person,
wherein the controller (18) is embodied to reconstruct a three-dimensional model data set of the procedure site by iterative reconstruction from the X-ray images (4),
wherein the controller (18) is furthermore embodied to reproject at least one two-dimensional supporting image (7), which corresponds to one of the recording geometries, by incorporating at least one item of further information, in order to determine and display the model data set supplemented by the further information, and
wherein the controller (18) is configured to insert, as an item of further information, a three-dimensional instrument model (6) into the model data set on the basis of a current item of position information of the instrument in the procedure site and to determine and display the projection of the instrument model (6) into supporting images (7) showing the recording geometries.

2. X-ray apparatus (13), having a controller (18) embodied for carrying out a method of image support for a person carrying out a minimally invasive procedure with an instrument in a procedure site of a patient,
wherein one two-dimensional X-ray image (4) respectively of the procedure site is recorded by means of the X-ray apparatus (13) in at least two recording geometries implementing different projection directions and chosen specific to the procedure, in particular by the person,
wherein the controller (18) is embodied to reconstruct a three-dimensional model data set of the procedure site by iterative reconstruction from the X-ray images (4),
wherein the controller (18) is furthermore embodied to reproject at least one two-dimensional supporting image (7), which corresponds to one of the recording geometries, by incorporating at least one item of further information, in order to determine and display the model data set supplemented by the further information, and
- wherein the controller (18) is configured, in at least two selection points (9) chosen as the selection image (8) by the user in one of the X-ray images (4) or a supporting image (7), to determine one three-dimensional position (12) respectively of the selection point (9) in the model data set by searching along a beam (10), defined in the recording geometry of the selection image (8), in which the selection points (9) were chosen, which beam crosses the selection point (9), for a jump in intensity in at least one search direction (11) in the model data set, the position of which is chosen as the three-dimensional position (12) of the selection point (9), and
- to determine at least one supporting image (7), reprojected with the selection points (9) as further information in a different recording geometry to that of the selection image (8), in which the selection points (9) were chosen.

3. X-ray apparatus according to claim 1 or 2, wherein less than 10, in particular less than 5, recording geometries chosen specific to the procedure are used.

4. X-ray apparatus according to one of claims 1 to 3, wherein the item of position information can be determined by a position-determining system registered with the X-ray apparatus (13) and/or from a two-dimensional localising image of the X-ray apparatus (13).

5. X-ray apparatus according to claim 4, wherein when using a single localising image, the three-dimensional item of position information is determined on the basis of at least one marker and/or the shaping of the instrument.

6. X-ray apparatus according to one of claims 1 to 5, wherein in the case of an instrument located in the procedure site as early as during recording of at least some of the X-ray images (4), the controller (18) is configured to segment and remove it in the X-ray images (4) and/or model data set.

7. X-ray apparatus according to one of claims 2 to 6, wherein directions and/or beam directions, perpendicular to the beam (10), of the recording geometry of at least the supporting image (7) or selection image (8), preferably of all recording geometries, through a point of the beam (10) currently being examined, are used as search directions (11).

8. X-ray apparatus according to one of claims 2 to 7, wherein the controller (18) is configured to adjust the three-dimensional position (12) of the selection point (9) as a function of a user-side shift of the projected selection point (9) in the supporting image (7) along the associated beam (10) projected into the supporting image (7).

9. X-ray apparatus according to one of claims 2 to 8, wherein at least one measure of a geometric form spanned by the selection points (9) can be determined and output on the basis of the three-dimensional positions (12) of the selection points (9), in particular in the case of two selection points (9), a distance between the selection points (9).

10. X-ray apparatus according to one of the preceding claims, wherein when a current X-ray image (4) is rerecorded, the controller (18) is configured to update the model data set in at least one of the recording geometries, in particular after 2D-3D registering of the model data set and the current X-ray image (4).

11. Computer program, which can be loaded into a storage device of the controller in order to configure a controller of an X-ray apparatus according to one of the preceding claims.

12. Electronically readable data carrier on which a computer program according to claim 11 is stored.

## Revendications

1. Dispositif (13) de rayon X, comportant un dispositif (18) de commande constitué pour le soutien par l'image d'une personne effectuant une intervention mini-invasive avec un instrument dans une partie d'intervention d'un patient,
dans lequel au moyen du dispositif (13) de rayon X respectivement une image (4) de rayon X en deux dimensions de la partie d'intervention peut être enregistrée dans des géométries d'enregistrement sélectionnées, en particulier par la personne, spécifiquement à l'intervention, réalisées dans au moins deux directions de projection différentes,
dans lequel le dispositif (18) de commande est constitué, pour reconstruire par reconstruction ou par itération à partir des images (4) de rayon X, un ensemble de données modèle en trois dimensions de la partie d'intervention,
dans lequel le dispositif (18) de commande est constitué en outre pour reprojeter avec incorporation d'au moins une information supplémentaire au moins une image (7) de soutien en deux dimensions, qui correspond à l'une des géométries d'enregistrement, afin de déterminer et de représenter l'ensemble de données modèle complété de l'information supplémentaire, et
dans lequel le dispositif (18) de commande est agencé pour insérer, dans l'ensemble de données modèle, un modèle (6) d'instrument en trois dimensions comme information supplémentaire sur la base d'une information de position en cours de l'instrument dans la partie d'intervention et pour déterminer la projection du modèle (6) d'instrument dans les images (7) (7) d'examen montrant des géométries d'enregistrement.

2. Dispositif (13) de rayon X, comportant un dispositif (18) de commande constitué pour le soutien par l'image d'une personne effectuant une intervention mini-invasive avec un instrument dans une partie d'intervention d'un patient,
dans lequel au moyen du dispositif (13) de rayon X respectivement une image (4) de rayon X en deux dimensions de la partie d'intervention peut être enregistrée dans des géométries d'enregistrement sélectionnées, en particulier par la personne, spécifiquement à l'intervention, réalisées dans au moins deux directions de projection différentes,
dans lequel le dispositif (18) de commande est constitué, pour reconstruire, par reconstruction ou par itération à partir des images (4) de rayon X, un ensemble de données modèle en trois dimensions de la partie d'intervention,
dans lequel le dispositif (18) de commande est constitué en outre pour reprojeter avec incorporation d'au moins une information supplémentaire au moins une image (7) de soutien en deux dimensions, qui correspond à l'une des géométries d'enregistrement, afin de déterminer et de représenter l'ensemble de données modèle complété de l'information supplémentaire, et
dans lequel le dispositif (18) de commande est agencé
- pour, dans au moins deux points (9) de sélection, sélectionnés par l'utilisateur dans l'une des images (4) de rayon X ou dans une image (7) de soutien comme image (8) de sélection, déterminer respectivement une position (12) en trois dimensions du point (9) de sélection dans l'ensemble de données modèle en recherchant un saut d'intensité dans au moins une direction (11) de recherche dans l'ensemble de données modèle le long d'un rayon (10) défini dans la géométrie d'enregistrement de l'image (8) de sélection, dans laquelle les points (9) de sélection ont été sélectionnés, rayon qui passe par le point (9) de sélection, dont la position est choisie comme position (12) en trois dimensions du point (9) de sélection, et
- pour déterminer au moins une image (7) de soutien reprojetée par les points (9) de sélection comme information supplémentaire dans une autre géométrie d'enregistrement que celle de l'image (8) de sélection, dans laquelle les points (9) de sélection ont été sélectionnés.

3. Dispositif de rayon X suivant la revendication 1 ou 2, dans lequel moins de 10, en particulier moins de 5 géométries d'enregistrement sélectionnées spécifiquement à l'intervention, sont utilisées.

4. Dispositif de rayon X suivant l'une des revendications 1 à 3, dans lequel l'information de position peut être déterminée par un système de détermination de position enregistrée par le dispositif (13) de rayon X et/ou à partir d'une image de localisation en deux dimensions du dispositif (13) de rayon X.

5. Dispositif de rayon X suivant la revendication 4, dans lequel, lorsque l'on utilise une seule image de localisation, la détermination de l'information de position en trois dimensions s'effectue sur la base d'au moins un marqueur et/ou de la forme donnée à l'instrument.

6. Dispositif de rayon X suivant l'une des revendications 1 à 5, dans lequel le dispositif (18) de commande est agencé pour segmenter et éliminer, déjà à la réception d'au moins une partie des images (4) de rayon X dans l'instrument se trouvant dans la partie d'intervention, cet instrument dans les images (4) de rayon X et/ou dans l'ensemble de données modèle.

7. Dispositif de rayon X suivant l'une des revendications 2 à 6, dans lequel sont utilisées comme directions (11) de recherche, des directions perpendiculaires au rayon (10) et/ou des directions de rayon de la géométrie d'enregistrement au moins de l'image (7) de soutien ou de l'image (8) de sélection, de préférence de toutes les géométries d'enregistrement passant par un point en examen en cours du rayon (10).

8. Dispositif de rayon X suivant l'une des revendications 2 à 7, dans lequel le dispositif (18) de commande est agencé pour adapter la position (12) en trois dimensions du point (9) de sélection en fonction d'un décalage de la part de l'utilisateur du point (9) de sélection projeté dans l'image (7) de soutien le long du rayon (10) associé projeté dans l'image (7) de soutien.

9. Dispositif de rayon X suivant l'une des revendications 2 à 8, dans lequel sur la base des positions (12) en trois dimensions des points (9) de sélection au moins une mesure d'une forme géométrique passant par les points (9) de sélection peut être déterminée et donnée, en particulier dans le cas de deux points (9) de sélection une distance entre les points (9) de sélection.

10. Dispositif de rayon X suivant l'une des revendications précédentes, dans lequel le dispositif (18) de commande est agencé pour mettre à jour l'ensemble de données modèle, lors du réenregistrement d'une image (4) de rayon X en cours dans au moins l'une des géométries d'enregistrement, en particulier après un enregistrement 2D-3D de l'ensemble de données modèle et de l'image (4) de rayon X en cours.

11. Programme d'ordinateur, qui peut, pour l'établissement d'un dispositif de commande d'un dispositif de rayon X suivant l'une des revendications précédentes, être chargé dans la mémoire du dispositif de commande.

12. Support de données déchiffrable électroniquement, sur lequel un programme d'ordinateur suivant la revendication 11 est mis en mémoire.
